# EUROPÄISCHE PATENTANMELDUNG

(11) **EP 0 681 850 A2**
(43) Veröffentlichungstag der Anmeldung: **15.11.1995**
(21) Anmeldenummer: 95250111.2
(22) Anmeldetag: 09.05.1995
(51) Int. Cl.: A61N 1/368

(54) **Verfahren zur Vermeidung des zeitlichen Zusammenfallens von stimulierten und spontanen Herzreaktionen**

(30) Priorität: 09.05.1994 DE 4416779
(71) Anmelder: BIOTRONIK Mess- und Therapiegeräte GmbH & Co Ingenieurbüro Berlin, D-12359 Berlin (DE)
(72) Erfinder: Fröhlich, Ronald, D-91056 Erlangen (DE); Bolz, Armin, D-91058 Erlangen (DE); Schaldach, Max, D-91054 Erlangen (DE)
(74) Vertreter: Christiansen, Henning, Dipl.-Ing.

(57) **Zusammenfassung**

Verfahren zur Vermeidung des zeitlichen Zusammenfallens von stimulierten und spontanen Herzaktionen bei der Aussendung von Herzreizimpulsen in den Vorhof oder die Kammer eines Herzens, wobei der Schritt der Aussendung des Herzreizimpulses derart zeitlich gesteuert ausgeführt wird, daß das gleichzeitige oder überlagerte Auftreten einer spontanen Herzaktion und eines evozierten Potentials verhindert ist, und im Falle einer atrium-synchronen Stimulation die Aussendung eines Herzreizimpulses im Ventrikel die Verzögerung zwischen der spontanen Atrium-Aktion und der Stimulation im Ventrikel kleiner als die natürliche AV-Überleitungszeit eingestellt wird und/oder im Falle der ventrikelinhibierten Stimulation in einer ersten Stufe eine Folge mehrerer Herzreizimpulse mit einer ersten Rate, anschließend in einer zweiten Stufe eine zweite Folge von Herzreizimpulsen mit einer zweiten, gegenüber der ersten im wesentlichen sprunghaft vergrößerten Rate und anschließend in einer dritten Stufe eine dritte Folge von Herzreizimpulsen mit einer dritten, gegenüber der zweiten im wesentlichen sprunghaft wieder verringerten Rate ausgesandt wird, wobei während mindestens der ersten mit der dritten Rate abgegebenen Herzreizimpulse das gleichzeitige oder überlagerte Auftreten einer spontanen Herzaktion und eines evozierten Potentials verhindert ist.

## Beschreibung

Die Erfindung betrifft ein Verfahren der im Oberbegriff des Anspruchs 1 angegebenen Art sowie eine Vorrichtung zur Durchführung des Verfahrens.

Die Erkennung der Art von Herzaktionen, d.h. die Unterscheidung stimulierter von spontanen bzw. autonomen Herzaktionen, ist für die Steuerung von Herzschrittmachern unter mehreren Gesichtspunkten belangvoll.

So ist bekannt, daß die Stimulationsschwelle ("threshold") eines Herzschrittmachers, d.h. die zur Erzeugung einer Erregung auf einen Reizimpuls hin nötige Reizimpulsenergie, sich im Zuge des Einwachsens der Schrittmacherelektrode(n), infolge des Einflusses von Pharmaka, durch pathologische Vorgänge im Herzen oder durch Mikrodislokationen der Elektrode(n) ändern kann. Eine einmal - etwa durch anfängliche Programmierung der Ausgangsspannung der Schrittmacherausgangsstufe - vorgenommene Einstellung der Reizimpulsenergie wird dann entweder im Hinblick auf die Sicherheit des Patienten oder auf einen batterieschonenden Betrieb des Schrittmachers und damit dessen Lebensdauer nicht mehr optimal sein.

Es ist daher ein wesentliches Leistungsmerkmal eines modernen Herzschrittmachersystems, daß bei Nachsorgeuntersuchungen die aktuelle Schwelle festgestellt und der Schrittmacher darauf neu eingestellt werden kann.

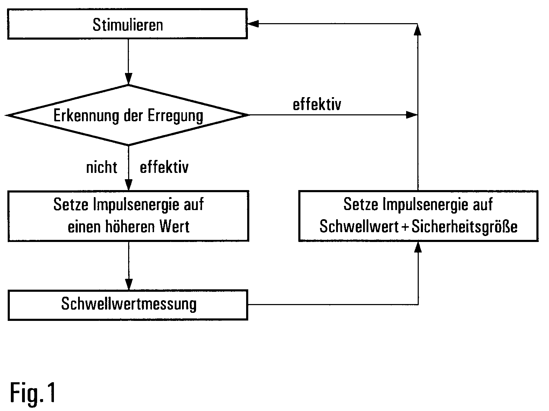

Soll eine solche Einstellung automatisch vorgenommen werden, so kann grundsätzlich nach dem in Fig. 1 gezeigten schematisierten Flußdiagramm vorgegangen werden.

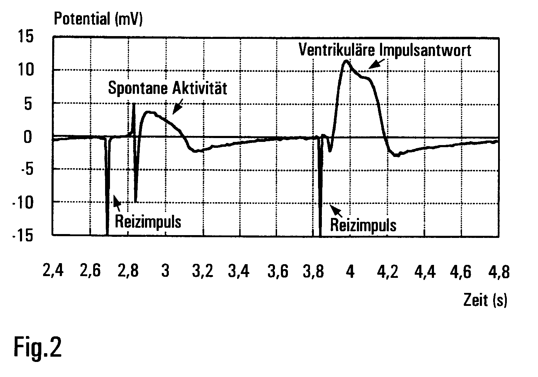

Hierfür ist es unbedingt erforderlich, stimulierte Herzaktionen (evozierte Potentiale) von spontanen bzw. autonomen Herzaktionen - beide sind beispielhaft in Fig. 2 dargestellt - ohne diagnostische Mitwirkung des Arztes zuverlässig zu unterscheiden, da es sonst zu lebensbedrohlichen Fehleinstellungen des Schrittmachers kommen kann.

Bei einer aus der US 4 674 509 B1 bekannten Anordnung wird versucht, durch Aussendung von Impulspaaren, von denen ein Impuls mit Sicherheit eine Erregung bewirkt und der andere nicht, und eine eine Subtraktion der Impulsantwort-Signale umfassende Auswertung das auf einen wirksamen Impuls zurückzuführende Herzaktionspotential von Artefakt-Anteilen zu isolieren. Eine Unterscheidung von konkurrierend auftretenden spontanen Herzaktionen ist damit nicht möglich, und die Anordnung ist als Hilfsmittel für die Diagnose und manuelle Einstellung der Impulsenergie durch den Arzt gedacht.

In der US 4 858 610 B1 ist eine Vorrichtung zum automatischen Nachweis evozierter Potentiale beschrieben, bei der zu deren Erfassung eine gesonderte Ringelektrode und Eingangsstufe vorgesehen ist. Die Erfassung der natürlichen bzw. spontanen Herzaktivität erfolgt in einem bipolaren Modus mittels dieser Ringelektrode und der Stimulationselektrode, allerdings lediglich, um ggfs. eine Inhibition des Reizimpulses zu bewirken und nicht zum Zwecke der Unterscheidung zwischen spontanen und stimulierten Herzaktionen. Bei dieser Vorrichtung wird über die Wahl eines Meßzeitfensters ein Ausblenden natürlicher Herzaktionen versucht. Fallen jedoch spontane Herzaktionen in das Zeitfenster - was keineswegs auszuschließen ist - muß es zu Fehlinterpretationen kommen. Das System ist daher für eine automatische Schwellwertbestimmung und Impulsenergieeinstellung ungeeignet.

Weiterhin ist es im Rahmen der Steuerung eines Herzschrittmachers durch aus Aktionen des autonomen Nervensystems gewonnene Signale - des sogenannten ANS-gesteuerten Schrittmachers, wie etwa beschrieben in M. Schaldach, Electrotherpy of the Heart - Technical Aspects in Cardiac Pacing, Springer Verlag 1992, S. 111 bis 120 von zentraler Bedeutung, unverfälscht autonome (d.h. von Stimulationsreaktionen freie) Signale verfügbar zu haben.

In den genannten sowie weiteren Anwendungsfällen führt die Möglichkeit des im wesentlichen gleichzeitigen, d.h. zeitlich überlagerten Auftretens spontaner und stimulierter Herzaktionen zu kaum lösbaren Problemen, weil sich dabei Signale mit Misch-Kurvenformen ergeben, die eine Klassifizierung der aufgenommenen Herzaktion als spontane oder stimulierte Aktion und eine entsprechende Auswertung unmöglich machen. Bei der Messung der Reizschwelle führt das Auftreten eines aufgrund der Kurvenform fälschlich als spontane Herzaktion klassifizierten "fusion-beats" beispielsweise dazu, daß die der spontanen Herzaktion überlagerte Stimulationsantwort nicht bemerkt und die Impulsenergie des Scherittmachers (unnötigerweise) heraufgesetzt oder gar in einen Notbetrieb übergegangen wird, obwohl das Herz durch die Stimulation bei der ursprünglich eingestellten Energie korrekt erregt worden war.

Der Erfindung liegt daher die Aufgabe zugrunde, ein Verfahren der eingangs genannten Gattung anzugeben, mit dessen Hilfe bei der Registrierung von Herzaktionspotentialen eine Erfassung von von Stimulationsreaktionen freien autonomen Herzaktionen einerseits bzw. reiner stimulierter Herzaktionen andererseits und somit eine sichere Unterscheidung spontaner bzw. autonomer von stimulierten Herzaktionen aufgrund ihrer Kurvenform vorgenommen werden kann. Weiterhin soll eine Vorrichtung zur Durchführung des Verfahrens angegeben werden.

Diese Aufgabe wird durch ein Verfahren mit den Merkmalen des Anspruchs 1 sowie eine Vorrichtung nach Anspruch 15 gelöst.

Die Erfindung schließt den Gedanken ein, das im wesentlichen gleichzeitige Auftreten spontaner und stimulierter Herzaktionen - sogenannter "fusion beats" - durch eine geeignete zeitliche Steuerung des Schrittmacherbetriebes zu unterbinden.

In einer atrium-synchronen Betriebsweise des Schrittmachers (dem DDD- oder VDD-Modus) wird dazu eine unterhalb der natürlichen AV-Überleitungszeit liegende AV-Verzögerung (insbesondere gleich oder kleiner als 100 ms) für den Reizimpuls eingestellt und nur derjenige Teil des zeitlichen Verlaufes des Herzaktionspotentials zur Erkennung von dessen Art (spontan oder stimuliert) herangezogen, der vor Ablauf der natürlichen AV-Überleitungszeit liegt, so daß noch keine spontane Erregungskomponente übergeleitet worden und somit im Meßsignal enthalten sein kann.
im VVI-Modus wird, ausgehend von einer vorbestimmten Stimulationsrate, eine erste Folge von Reizimpulsen mit vergrößertem Impulsabstand (verringerter Rate) ausgesandt und anschließend der Impulsabstand wieder sprunghaft auf den ursprünglichen Wert verringert (die Rate erhöht) und eine zweite Folge von Reizimpulsen ausgesandt, wobei die Registrierung und Auswertung des Herzaktionspotentials innerhalb der letzteren Folge - vorzugsweise zu deren Beginn - erfolgt.

Nachdem auf diese Weise das Auftreten nicht klassifizierbarer Mischsignale verhindert wird, ist es möglich, eine Unterscheidung zwischen stimulierten und spontanen Herzaktionen primär aufgrund der sich unterscheidenden Signalform vorzunehmen. Unter Beachtung der typischen Signalformen (siehe Fig. 2), die sich in der Frequenzdomäne dadurch auszeichnen, daß das evozierte Potential (speziell die ventrikuläre Impulsantwort - VER) bei niedrigen Frequenzen Anteile mit hoher Amplitude hat, während die autonome Aktivität bei höheren Frequenzen Anteile mit hoher Amplitude hat, werden dazu vor allem folgende Vorgehensweisen vorgeschlagen:
a) eine zur Aufteilung des Gesamt-Frequenzspektrums in charakteristische Bereiche geeignete Filterung mit anschließender Amplitudendiskriminierung,
b) eine Frequenzanalyse (etwa durch schnelle Fourier-Transformation - FFT), speziell unter Einschluß eines Vergleiches mit vorgegebenen Mustern, oder
c) ein Sampling mit genügend vielen Meßpunkten in einem hinreichend breiten Meßzeitfenster mit anschließendem Vergleich mit einer vorgegebenen Referenzspannung.

Die Frequenzanalyse nach b) mit anschließender Bewertung kann einen Amplitudenvergleich der Signalanteile bei mindestens zwei vorbestimmten Frequenzen oder in mindestens zwei Frequenzbereichen einschließen.

Die Amplitudendiskriminierung nach c) kann eine Unterscheidung zwischen spontaner Herzaktion und evoziertem Potential anhand der absoluten oder relativen Häufigkeit des Auftretens von Amplitudenwerten unterhalb der Referenzspannung einschließen; es kann etwa schon das Auftreten eines unterhalb der Referenzspannung liegenden Signalwertes als Kennzeichen für das Vorliegen einer spontanen Herzaktion gelten.

Die Messungen erfolgen in einem Meßzeitfenster mit vorbestimmter Breite - nach Untersuchungen der Erfinder vorzugsweise etwa 60 ms - , dessen Beginn - vorzugsweise innerhalb des Bereiches von 150 bis 250 ms nach dem Stimulationsimpuls - programmierbar ist.

Die Abtastrate für die einzelnen Messungen innerhalb des Meßzeitfensters ist vorzugsweise größer als 100 Hz, und die Referenzspannung ist etwa zwischen 1 und 15 mV in Schritten von 1 mV einstellbar.

Zur Ausblendung von Signalanteilen des Reizimpulses wird eine Signalaustastung - vorzugsweise für 50 ms - und zur Ausfilterung von Störfrequenzanteilen vor der Auswertung eine Bandfilterung mit einem Durchlaßbereich von etwa 0,5 bis 10 Hz vorgenommen.

Im Ablauf eines Verfahrens zur automatischen Einstellung der Impulsenergie - speziell der Impulsamplitude bzw. Ausgangsspannung des Schrittmachers - als wichtigem Anwendungsfall der Erfindung werden in Abhängigkeit vom Betriebsmodus jeweils entsprechende Vorkehrungen zur Vermeidung der Entstehens von "fusion beats" getroffen.

Um Verfälschungen der Messungen durch "fusion beats" sowie die im Ausgangskondensator des Schrittmachers noch gespeicherte Ladung bei atrium-synchronem Betrieb zu vermeiden, wird bei Feststellung fehlender Effektivität des anfänglich ausgesandten Impulses eine unterhalb der natürlichen AV-Überleitungszeit liegende AV-Verzögerung (etwa 100 ms) eingestellt und vor dem folgenden Impuls mit erhöhter Impulsenergie zunächst mindestens ein weiterer Impuls mit der anfänglich eingestellten Energie ausgesandt und für diesen die Effektivität festgestellt.

Um einen korrekten Verfahrensablauf speziell auch bei Vorliegen eines 2:1-AV-Blockes zu sichern, wobei jede zweite ventrikuläre Impulsantwort inhibiert ist, wird bei Ausfall des ersten Impulses nach der Neueinstellung der AV-Verzögerung der nächstfolgende nicht-inhibierte Impuls abgewartet und dessen Effektivität festgestellt.

Neben der Ermittlung der Reizschwelle und - darauf basierend - der Einstellung der Impulsenergie können die mittels des Verfahrens gewonnenen, unverfälschten Herzaktionspotentiale zur Gewinnung von ANS-Informationen - etwa als Vergleichsgröße zu impedanzplethysmografisch gewonnenen Größen oder zur Festlegung der Zeitachse - verwendet werden.

Eine Vorrichtung zur Durchführung des Verfahrens weist Mittel zur Umschaltung der AV-Verzögerung bzw. Mittel zur Umschaltung der Stimulationsfrequenz zwischen vorbestimmten Werten - je nach Betriebsweise des Schrittmachers - sowie Mittel zur Steuerung des Verfahrensablaufes auf, die vorzugsweise in die Schrittmachersteuerung integriert sind.

Eine vorteilhafte Ausführung weist eine von der Schrittmachereingangsschaltung zur Erfassung eines intrakardialen EKG getrennte, vor der Anti-aliasing-Schaltung des Schrittmachereinganges mit der Aufnahmeelektrode verbundene Aufnahme- und Auswertungsschaltung auf, die vorzugsweise als eigenständige integrierte Schaltung ausgeführt und implantierbar ist, wobei sie von außerhalb des Körpers, insbesondere über einen Reed-Schalter, aktivierbar ist.

Die Aufnahme- und Auswertungsschaltung umfaßt insbesondere
- eine Austastschaltung zum Ausblenden von Signalen unmittelbar nach dem Herzreizimpuls,
- einen Vorverstärker
- einen programmierbaren Meßverstärker,
- ein Bandfilter mit einem Durchlaßbereich von 0,5 bis 10 Hz,
- eine Vergleichereinheit zur Amplitudendiskriminierung des Meßsignals,
- einen A/D-Wandler und
- eine logische Verarbeitungseinheit zur Analyse der Vergleichsergebnisse entsprechend dem Verfahrensablauf.

Die Steuerung der Vorrichtung kann mindestens teilweise über den Mikroprozessor des Schrittmachers erfolgen, wobei auch dessen Telemetriesystem zur Durchführung des Verfahrens sowie zur Sicherstellung der Informationsübermittlung an einen Arzt und von Eingriffsmöglichkeiten für diesen genutzt werden kann. Der separate Test-Schaltkreis ist dann über einen Testkanal mit dem Schrittmacher verbunden.

Um den Zeitabstand zum Stimulationsimpuls verfügbar zu haben, ist eine Signalverbindung zwischen Schrittmacher und Aufnahme- und Auswertungsschaltung zur Übermittlung eines die Stimulationsimpulse repräsentierenden Signals oder ein eigenständiger Meßkanal zur Erfassung der Stimulationsimpulse vorgesehen. Weiterhin ist eine Einrichtung zur Triggerung des Verfahrensablaufes in Reaktion auf die erfaßten Stimulationsimpulse vorgesehen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein schematisches Flußdiagramm eines Verfahrensablaufes zur automatischen Einstellung der Impulsenergie bei einem Schrittmacher,
Figur 2 eine grafische Darstellung eines ventrikulären Elektrogramms, das eine spontane Herzaktion und ein evoziertes Potential in Abhängigkeit von der Zeit zeigt,
Figur 3 eine grafische Darstellung eines evozierten Potentials in Abhängigkeit von der Zeit mit einem Meßzeitfenster für die Effektivitätserkennung,
Figuren 4a bis 4c ein Flußdiagramm eines Verfahrens zur automatischen Einstellung einer Schrittmacherimpulsamplitude im DDD-Modus eines Herzschrittmachers gemaß einer Ausführungsform der Erfindung,
Figuren 5a bis 5d ein Flußdiagramm eines Verfahrens zur automatischen Einstellung einer Schrittmacherimpulsamplitude im VVI-Modus eines Herzschrittmachers gemäß einer weiteren Ausführungsform der Erfindung,
Figur 6 eine schematische Darstellung der Rate bei einem Stimulations- und einem spontanen Herzrhythmus in Abhängigkeit von der Zeit bei einer Ausgestaltung des Verfahrens nach Fig. 5a bis 5c,
Figur 7 ein vereinfachtes Blockschaltbild einer Vorrichtung zur Durchführung des erfindungsgemäßen Verfahrens in einer Ausführungsform sowie einer Schrittmachereingangsschaltung und
Figur 8 eine schematische Darstellung des Zusammenwirkens einer Vorrichtung zur Durchführung des erfindungsgemäßen Verfahrens mit einem programmierbaren Herzschrittmacher.

In Fig. 1 ist schematisch ein Verfahrensablauf für die automatische Einstellung der Impulsenergie eines Stimulators für reizbares Körpergewebe - beispielsweise eines Herzschrittmachers - dargestellt.

Das Verfahren beginnt, indem mit einer voreingestellten Energie stimuliert und anschließend festgestellt wird, ob die Stimulation zu einer Erregung geführt hat oder nicht. War sie effektiv, wird weiterhin mit der eingestellten Energie stimuliert. War sie nicht effektiv, wird die Impulsenergie auf einen (wesentlich) höheren Wert gesetzt und anschließend eine (weiter unten genauer erläuterte) Prozedur zur Bestimmung der Reizschwelle ausgeführt. Der im Ergebnis dieser Prozedur erhaltene Schwellwert wird mit einer Sicherheitsgröße (in der Figur einem Summanden) verarbeitet und so der Wert erhalten, auf den die Impulsenergie für die weitere Stimulierung eingestellt wird.

Der beispielhaft dargestellte Ablauf ist im Ansatz sicherheitsorientiert, d.h. solange eine Stimulation mit dem voreingestellten Energiewert zu einer Erregung führt, wird mit diesem Energiewert weitergearbeitet, ohne eine mögliche Senkung der Impulsenergie zu prüfen. Steht bei der Einstellung die Minimierung des Batterieverbrauchs und damit die Maximierung der Lebensdauer im Mittelpunkt, ist der Ablauf entsprechend zu modifizieren.

Fig. 2 zeigt beispielartig ein intraventrikulär aufgenommenes Elektrogramm, in dessen linkem Bereich ein Stimulationsimpuls mit sich anschließender autonomer bzw. spontaner Herzaktion und in dessen rechtem Bereich ein Stimulationsimpuls mit auf diesen folgender Impulsantwort (evoziertem Potential) in Abhängigkeit von der Zeit gezeigt ist.

Es ist zu erkennen, daß die autonome Herzaktion ein Signal mit sich deutlich vom evoziertem Potantial unterscheidender Signalform liefert, wobei insbesondere die starke Schwankung der Amplitude im Einsetzbereich der spontanen Herzaktion ins Auge fällt.

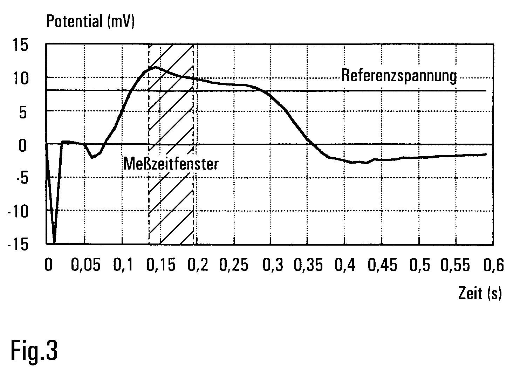

Fig. 3 bezeichnet mit der Angabe eines (schraffierten) Zeitfensters einen Abschnitt auf der - mit anderem Maßstab als in Fig. 2 dargestellten - Zeitachse, der zur Feststellung von Unterschieden in der Signalform der beiden in Fig. 2 gezeigten Signale besonders geeignet ist.

Bei der nachfolgenden Beschreibung von Ausführungsbeispielen wird davon ausgegangen, daß in diesem Zeitfenster mit einer Breite von 60 ms und im Bereich von knapp unter 150 ms bis 250 ms (in der Figur auf ca. 130 ms eingestellt) nach einem Stimulationsimpuls ein Sampling der an der Spitze einer Schrittmacherleitung aufgenommenen, einer Bandfilterung mit einem Durchlaßbereich von 0,5 bis 10 Hz unterworfenen, Signale mit einer Abtastfrequenz von minimal 100 Hz erfolgt. Die erhaltenen Spannungswerte werden einer Amplitudendiskriminierung mit einer im Bereich von 1 bis 15 mV einstellbaren Referenzspannung unterzogen. Eine Stimulation wird als erfolgreich (als Erregung - "capture") angesehen, wenn alle im Zeitfenster aufgenommenen Spannungswerte über der programmierten Referenzspannung liegen, hingegen als nicht erfolgreich ("no capture"), wenn mindestens einer der Werte unterhalb der Referenzspannung liegt.

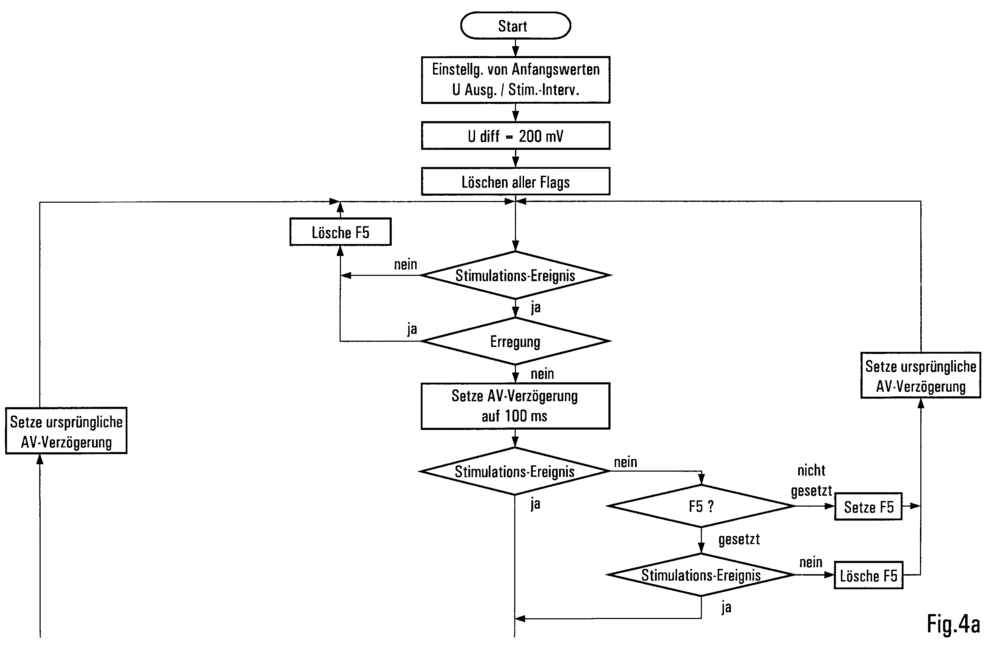

In den Figuren 4a bis 4c ist das Flußdiagramm eines Verfahrens zur automatischen Einstellung der Schrittmacherimpulsamplitude im DDD-Modus eines Herzschrittmachers gemäß einer Ausführungsform der Erfindung gezeigt. Das Verfahren wird nachfolgend kurz beschrieben, ohne daß alle aus dem Diagramm ohne weiteres ersichtlichen Schritte nochmals im einzelnen wiederholt werden. Im Programmablauf werden vier Kennzeichen (Flags) verwendet.

Zu Beginn werden vom Arzt Anfangsparameter (insbesondere eine Ausgangsspannung und eine Impulsrate und ein Spannungsdifferenzbetrag, um den die Ausgangsspannung in den anchfolgenden Schritten jeweils zu verringern ist - in Fig. 4a mit U diff = 200 ms angegeben) programmiert, der Programmiermagnet wird entfernt, und es werden alle Flags (die nachfolgend bzw. im Diagramm erläutert sind) gelöscht.

Wird auf ein Stimulations-Ereignis hin eine Erregung nachgewiesen, wird die Schleife geschlossen, und der Schrittmacher arbeitet mit den programmierten Parametern.

Wird keine Erregung nachgewiesen, wird die AV-Verzögerung auf 100 ms eingestellt, um - wie weiter oben erläutert - "fusion-beats" auszuschließen, und unter Zuhilfenahme des Flag F5 wird ein Teilablauf abgearbeitet, der zur Vermeidung von Fehlinterpretationen bei möglichem Vorliegen eines 2:1-AV-Blocks dient. (Andere Arten von AV-Blöcken, etwa der 4:1-Block, sind nicht lebensbedrohlich, so daß daraus resultierende Fehlaussagen unkritisch sind.) Dazu wird das Flag F5 gesetzt, wenn auf einen ersten Impuls keine Erregung festgestellt wurde und der nächste Impuls inhibiert ist. Daraufhin wird im Algorithmus der Impuls unmittelbar nach dem inhibierten daraufhin untersucht, ob er von einer Erregung gefolgt ist oder nicht. Wenn ja, wird - nach Löschen von F5 - wieder die ursprüngliche AV-Verzögerung eingestellt und der vorhergehende Ablauf wiederholt.

Wenn nein, wird die Ausgangsspannung auf einen ersten Sicherheitswert U Ausg von 4,8 V und danach, falls noch immer keine Erregung erreicht wurde, auf 9,6 V heraufgesetzt.

Für eine vom Arzt vorgenommene Messung der Reizschwelle ist hier ein gesonderter Zugang vorgesehen, über den - unter Setzung eines Flag F4 - die Ausgangsspannung getrennt vom übrigen Ablauf programmiert und in 100-mV-Schritten variiert werden kann.

Im Programmablauf wird dann wieder die ursprüngliche AV-Verzögerung eingestellt und es werden zehn Ereignisse beim (ersten oder zweiten) Sicherheits-Amplitudenwert abgewartet. Dann wird mit jeweils um die vorgegebene Differenzspannung U diff abgesenkter Amplitude U test in der entsprechend der Lage der Reizschwelle erforderlichen Anzahl von Durchläufen der eigentliche Reizschwellentest ausgeführt. Dabei werden die weiteren Flags F2 und F3 eingesetzt. wobei mit F2 das Abwarten jeweils zweier erfolgreicher Nachweiszyklen bei jedem Wert der Testamplitude vor einer weiteren Reduzierung und mit F3 das Abwarten zweier nicht mehr erfolgreicher Nachweiszyklen vor der Fixierung der aktuellen Amplitude als Reizschwelle realisiert wird.

Nach Abschluß des Reizschwellentests wird mit der um 1V erhöhten letzten Testspannung als aktueller Ausgangsspannung weiterstimuliert. Falls der Ablauf einer Untersuchung durch den Arzt folgte (Flag F4 gesetzt), wird der Ablauf über einen separaten Ausgang unter Einstellung von U Ausg auf 4,8 V beendet.

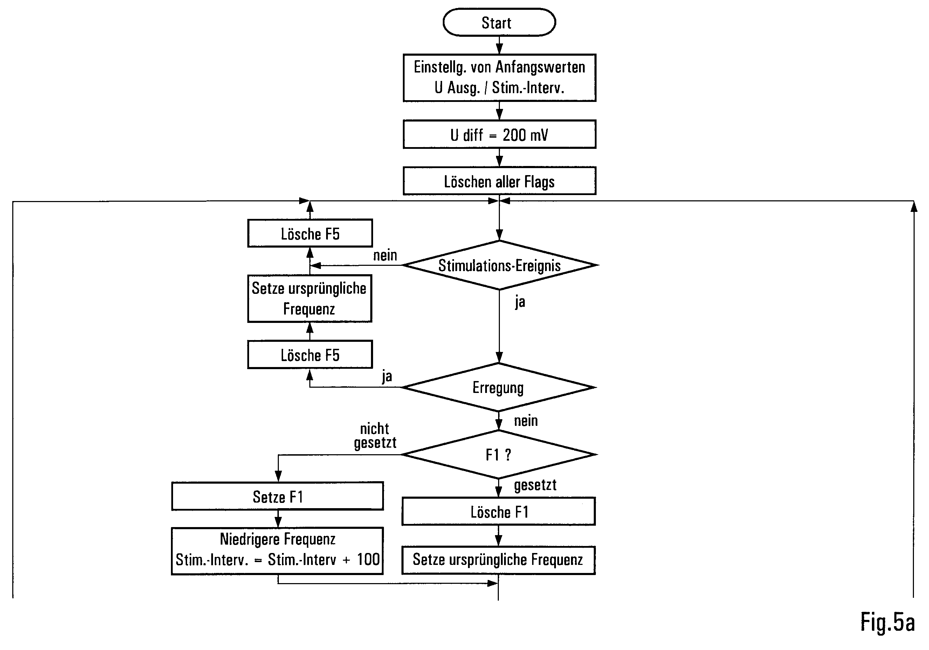

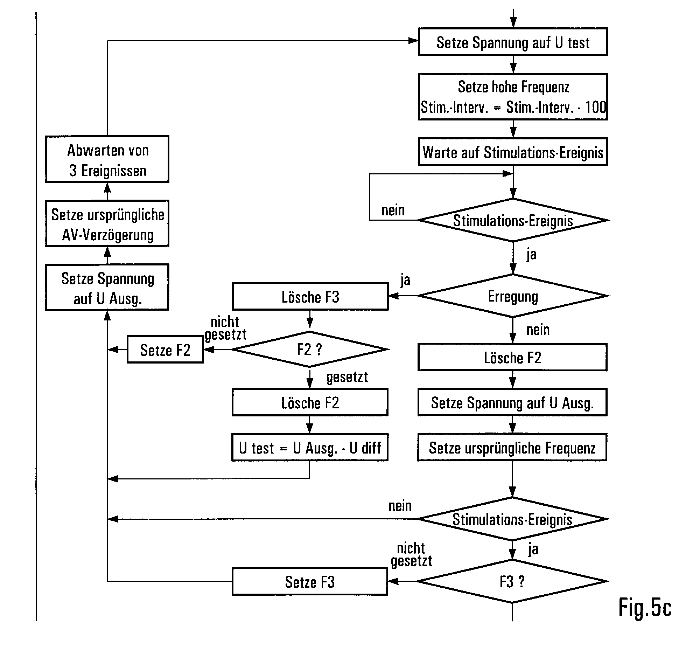

In den Figuren 5a bis 5c ist das Flußdiagramm eines zu dem in Fig. 4a bis 4c gezeigten Verfahren ähnlichen Verfahrens im VVI-Modus eines Herzschrittmachers gezeigt. Nachfolgend wird nur auf die Unterschiede dieses Verfahren gegenüber dem vorbeschriebenen hingewiesen, wobei auch hier die aus dem Diagramm ersichtlichen Schritte nicht im einzelnen wiederholt werden. Im Programmablauf werden fünf Kennzeichen (Flags) verwendet.

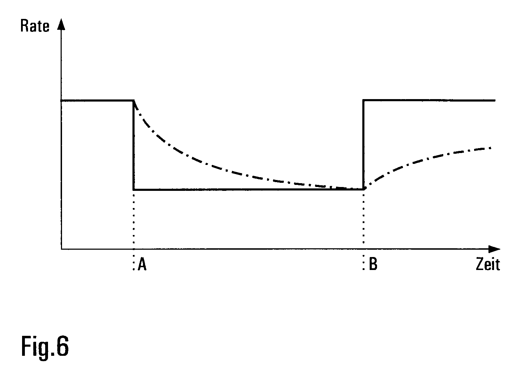

Wird auf ein Stimulationsereignis hin keine Erregung nachgewiesen, wird zur Realisierung eines weiter unten unter Bezugnahme auf Fig. 6 genauer erläuterten Vorgehens unter Zuhilfenahme des Flag F1 die Stimulationsfrequenz zunächst verringert und dann wieder auf den ursprünglichen Wert erhöht, um - wie weiter oben für diesen Betriebsmodus bereits erwähnt - "fusion-beats" auszuschließen, und unter Zuhilfenahme der Flag F5 werden wieder Fehlinterpretationen bei möglichem Vorliegen eines 2:1-AV-Blocks vermieden. (Dies wurde oben mit Variation der AV-Verzögerung beschrieben und wird hier analog unter Variation der Stimulationsfrequenz bzw. -rate ausgeführt.)

Der weitere Ablauf entspricht dem Verfahren im DDD-Modus, wobei an die Stelle einer Variation der AV-Verzögerung jeweils eine solche der Stimulationsfrequenz tritt und zwischen die Schritte "Lösche F2" und "F3" im Hauptstrang die Wiedereinstellung der gewählten Spannung U Ausg und der ursprünglichen Frequenz sowie das Abwarten eines Stimulationsereignisses eingeschoben sind.

Fig. 6 zeigt in einer schematischen Darstellung, wie im VVI-Betriebsmodus - in dem bekanntlich nicht atriumsynchron stimuliert wird und es daher keine Möglichkeit gibt, das Zusammenfallen von stimulierten und spontanen Herzaktionen durch Verkürzung der AV-Verzögerung zu verhindern - ein solches Zusammenfallen ("fusion beats") verhindert werden kann:
Wenn bei einer bestimmten voreingestellten Stimulationsenergie und -rate keine Impulsantwort beobachtet wird, wird zu einem Zeitpunkt A die Stimulationsrate (in der Figur mit der durchgezogenen Linie dargestellt) um einen vorbestimmten Betrag verringert und mit der neuen Rate für einen bestimmten Zeitraum weiter stimuliert. Der Zeitraum wird so bemessen, daß die natürliche Sinusfrequenz (in der Figur mit einer strichpunktierten Linie bezeichnet) sich dem neuen Wert der Stimulationsrate anpassen kann. Danach wird zum Zeitpunkt B die Stimulationsrate wieder auf den ursprünglichen Wert heraufgesetzt. Da die natürliche Herzrate wiederum - wie in der Figur am langsamen Ansteigen der strichpunktierten Linie zu erkennen - vezögert nachfolgt, ist nach dem Heraufsetzen ein Zeitbereich von mehreren Impulsperioden verfügbar, in dem spontane Herzaktionen mit geringerer Frequenz als evozierte Potentiale auftreten, womit ein ständiges Zusammenfallen beider innerhalb des Meßzeitfensters sicher verhindert wird.

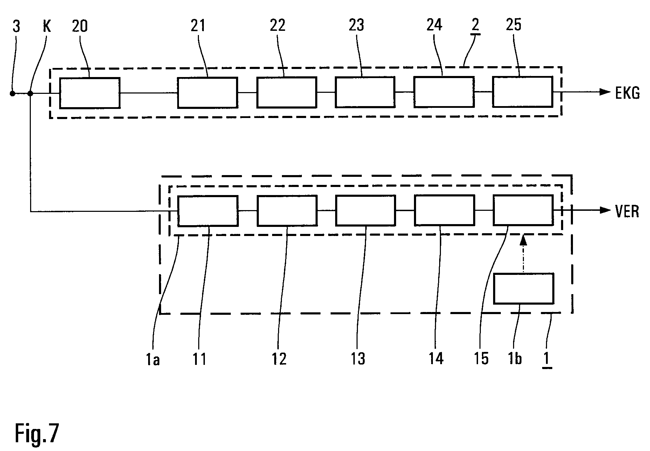

Fig. 7 ist ein vereinfachtes Blockschaltbild der wesentlichsten Elemente einer Vorrichtung 1 zur Durchführung des erfindungsgemäßen Verfahrens in einer Ausführungsform, gezeigt zusammen mit einer Schrittmachereingangsschaltung 2.

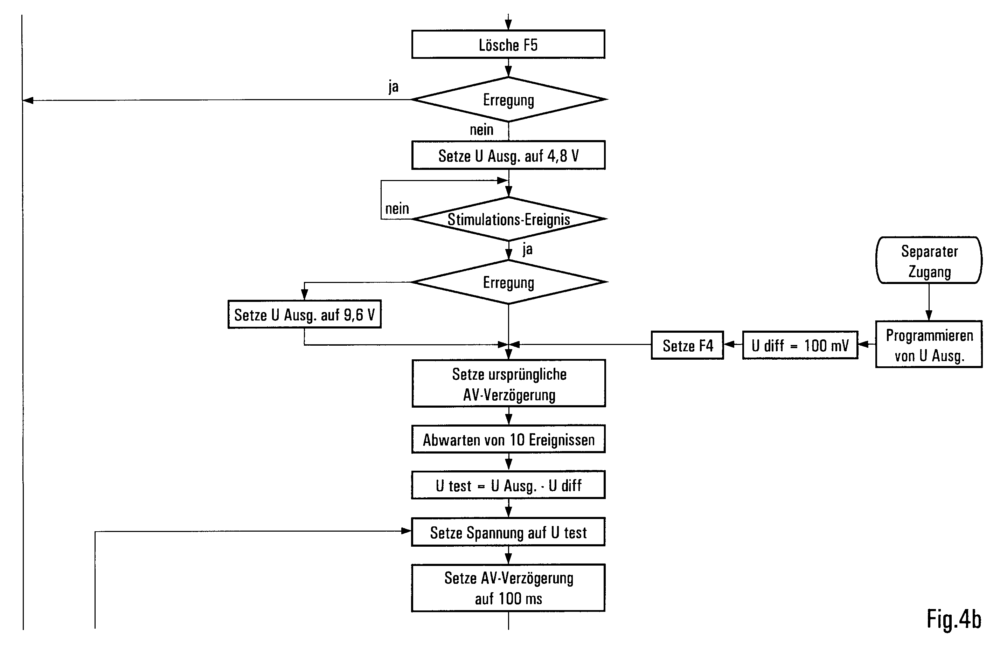

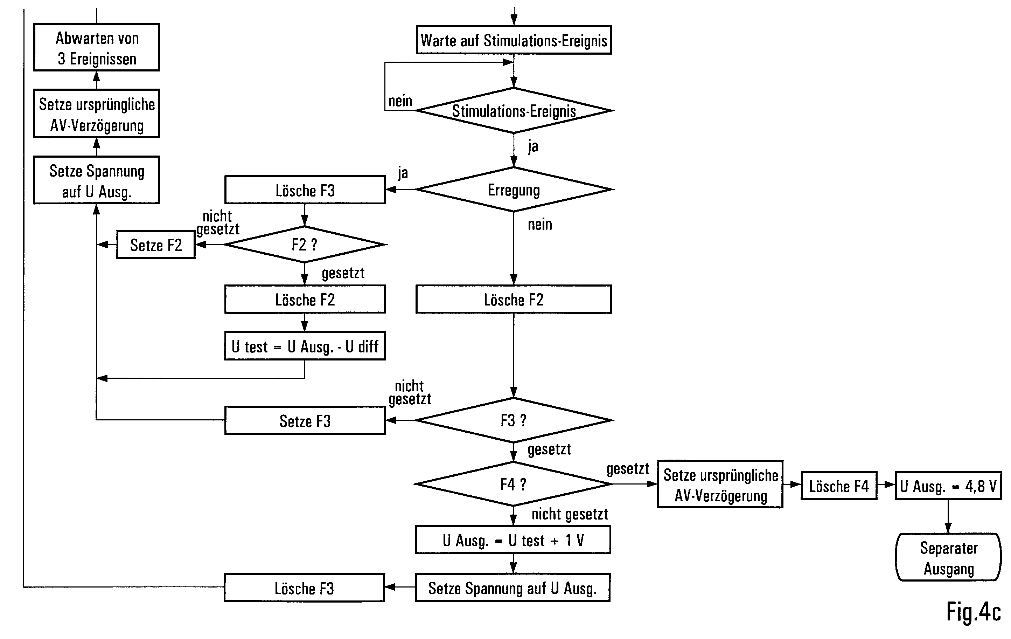

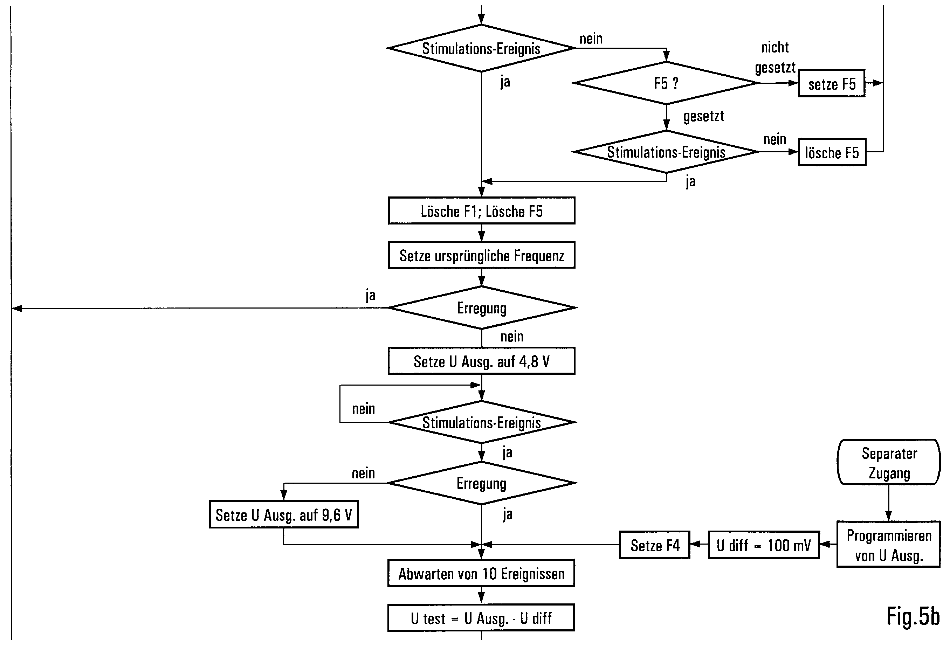

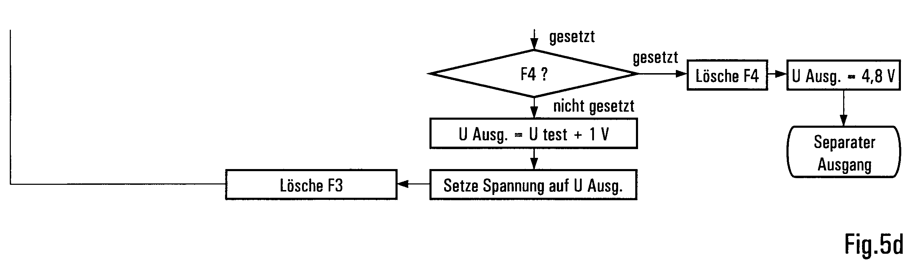

Die Vorrichtung 1 zur Effektivitätserkennung weist eine an einem Knoten K mit einer Schrittmacherleitung 3 verbundene Aufnahme- und Auswertungsschaltung 1a und eine Steuereinheit 1b auf. Die Aufnahme- und Auswertungsschaltung 1a weist einen Austast- bzw. Blankingschalter 11, dessen Austastperiode auf mindestens 50 ms eingestellt ist, einen mit dessen Ausgang verbundenen Vorverstärker 12, einen mit dessen Ausgang verbundenen Verstärker mit programmierbarer Verstärkung 13, ein mit dessen Ausgang verbundenes Bandfilter 14 mit einem Durchlaßbereich von etwa 0,5 bis 10 Hz und eine mit dessen Ausgang verbundene Diskriminator- bzw. Vergleichereinheit 15 auf, an deren Ausgang ein das Vorliegen oder Nichtvorliegen einer ventrikulären Impulsantwort kennzeichnendes Signal VER bereitsteht. Dieses ist entsprechend dem gewählten Verfahrensablauf - vgl. beispielsweise Fig. 4 oder Fig. 5 - (vorzugsweise in digitaler Form) weiterzuverarbeiten. Die Weiterverarbeitung wird ebenso wie der Betrieb der Eingangsstufe - darunter insbesondere die Einstellung des Meßzeitfensters und der Referenzspannung - von der Steuereinheit 1b gesteuert
Die Schrittmachereingangsstufe 2, deren Eingang ebenfalls über den Knoten K mit der Schrittmacherleitung verbunden ist, weist eine Anti-Aliasing-Schaltung 20, einen Austast- bzw. Blankingschalter 21, einen mit dessen Ausgang verbundenen Vorverstärker 22, einen mit dessen Ausgang verbundenen programmierbaren Verstärker 23, eine mit dessen Ausgang verbundene Filterstufe 24 mit den für eine EKG-Eingangssschaltung üblichen Filtergrößen und eine mit deren Ausgang verbundene Vergleichereinheit 25 auf, an deren Ausgang ein herkömmliches EKG-Signal ECG bereitsteht. Ihr Aufbau entspricht also äußerlich weitgehend demjenigen der separaten Eingangsstufe 1, durch andere Einstellung der Filter- und Verstärkungsparameter ergibt sich jedoch eine abweichende Funktion.

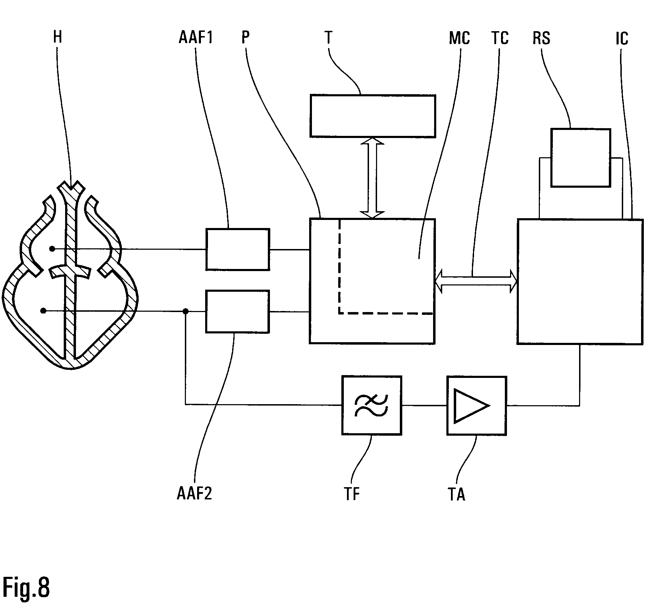

Figur 8 ist eine schematische Darstellung des Zusammenwirkens einer mit einer integrierten Schaltung IC realisierten Vorrichtung zur automatischen Einstellung der Impulsenergie ähnlich der Vorrichtung nach Fig. 7 mit einem programmierbaren Zweikammer-Herzschrittmacher P.

Der Schrittmacher P steht über je eine im Atrium und im Ventrikel eines Herzens H angeordnete Elektrode mit dem reizbaren Herzgewebe der jeweiligen Kammer in Verbindung und kann dort Reizimpulse anlegen sowie - jeweils über eine Anti-Aliasing-Einheit AAF1 bzw. AAF2 - intrakardiale Elektrogramme aufnehmen. Er weist eine Mikroprozessorsteuerung MC auf und steht weiterhin wahlweise über eine Telemetrieeinheit T mit einem behandelnden Arzt in Verbindung, der über diese Einheit eine Programmierung des Schrittmachers vornehmen und von diesem Daten erhalten kann.

Die Vorrichtung zur Effektivitätserkennung und automatischen Impulsenergieeinstellung ist in diesem Beispiel verteilt realisiert und umfaßt eine Filterbaugruppe TF, eine Verstärkerbaugruppe TA, die Auswertungs-Baugruppe IC und Funktionen der Schrittmacher-Steuereinheit MC, mit der die Auswertungs-Baugruppe IC über einen Testkanal TC verbunden ist. Über diesen werden Programmstrings vom Mikroprozessor MC und auch die Abgabe von Stimulationsimpulsen kennzeichnende Signale vom Schrittmacher P an den IC übertragen, wenn ein Reedschalter RS geöffnet und ein die Durchführung der automatischen Impulsenergieeinstellung kennzeichnendes Bit gesetzt ist.

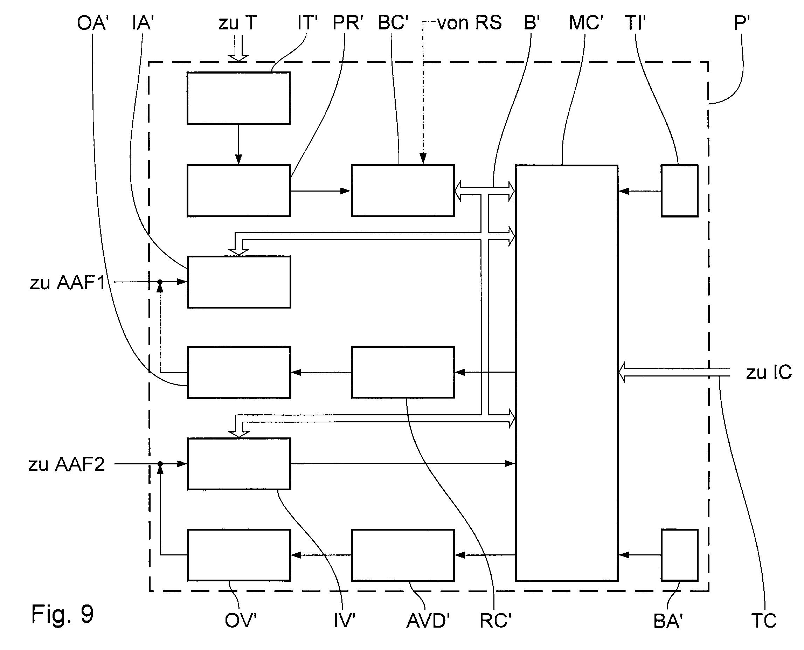

Figur 9 ist eine stark vereinfachte schematische Darstellung eines sowohl für atriumsynchronen (VDD, DDD) als auch für ventrikelinhibierten Betrieb (VVI) ausgebildeten ratenadaptiven Zweikammerschrittmachers P' mit ANS-Signalbasierter Ratensteuerung, der in einer die Erfindung ausführenden Gesamtanordnung die Stelle des Schrittmachers P in Fig. 8 einnehmen kann. Für die Umgebung des Schrittmachers sind daher in Fig. 9 dieselben Bezugszeichen verwendet wie in Fig. 8.

Der Schrittmacher P' weist eine Mikroprozessor-Steuereinheit MC', eine dieser zugeordnete Zeitbasis TI', eine Batterie BA' zur Stromversorgung aller Teile sowie eine Telemetrie-Empfangs- und Eingangsstufe IT', ein mit deren Ausgang verbundenes Programmregister PR' und eine mit dessen Ausgang verbundene Bussteuerung BC' für einen Systembus B' auf. Sie umfaßt weiterhin eine mit der (in Fig. 8 zu erkennenden) atrialen Sensing- und Stimulationselektrode verbundene Eingangsschaltung IA' für atriale EKG-Signale und eine mit der ventrikulären Sensing- und Stimulationselektrode verbundene Eingangsschaltung IV' für ventrikuläre EKG-Signale, deren Ausgänge jeweils mit der Steuereinheit MC' verbunden sind und die von dieser über den Systembus B' gesteuert werden. Schließlich umfaßt der Schrittmacher eine Ratensteuerschaltung RC', deren Ausgang mit einer Ausgangsstufe OA' zur Erzeugung atrialer Reizimpulse verbunden ist, sowie eine AV-Verzögerungseinheit AVD', deren Ausgang mit einer Ausgangsstufe OV' zur Erzeugung ventrikulärer Reizimpulse verbunden ist. Weitere übliche Komponenten - wie ein EOL-Indikator, eine Stufe zum Runaway-Schutz etc. - , die mit der Ausführung der Erfindung nicht in engerem Zusammenhang stehen, wurden zur Vereinfachung weggelassen. Zu beachten ist, daß die Steuerung MC' über den Datenbus TC auch mit der in FIg. 8 gezeigten und oben beschriebenen Bauteilgruppe TF-TA-IC verbunden ist und von dort Daten empfängt, die die Klassifizierung aufgenommener Herzaktionen betreffen.

Der Betrieb dieses Schrittmachers erfolgt entsprechend den oben ausgeführten Verfahrensmerkmalen. Zur Gewinnung von dem autonomen Nervensystem entspringenden Ratensteuerinformationen aus dem intrakardialen EKG (ANS-Informationen) wird speziell - je nach Betriebsmodus durch Verringerung der AV-Verzögerung auf kleiner oder gleich 100 ms oder das oben unter Bezugnahme auf Fig. 6 beschriebene Ratensteuerprogramm - über die Steuereinheit MC' und die Ratensteuerschaltung RC' bzw. die AV-Verzögerungseinheit AVD' die Reizimpulsfolge so eingetellt, daß mindestens über einige Impulsintervalle das Auftreten einer überlagerten spontanen und stimulierten Herzaktion auszuschließen ist. Zur Auswertung der ANS-Information steht mithin eine eindeutig zuordenbare Kurvenform zur Verfügung.

Der entsprechende Meßprogrammablauf steht im Programmregister PR' bereit und wird auf ein Schaltsignal vom (in Fig. 8 gezeigten) Reedschalter RS initiiert. Nach der Gewinnung der benötigten Meßwerte kann dann normal mit den aktuellen Stimulationsparametern weiterstimuliert werden, wobei ggfs. im Ergebnis der Messung eine neue Einstellung der Ratensteuerschaltung RC' vorgenommen wurde.

Die Erfindung beschränkt sich in ihrer Ausführung nicht auf die vorstehend angegebene bevorzugten Ausführungsbeispiele. Vielmehr ist eine Anzahl von Varianten denkbar, welche von der dargestellten Lösung auch bei grundsätzlich anders gearteten Ausführungen Gebrauch macht.

## Patentansprüche

1. Verfahren zur Vermeidung des zeitlichen Zusammenfallens von stimulierten und spontanen Herzaktionen bei der Aussendung von Herzreizimpulsen in den Vorhof oder die Kammer eines Herzens,
**dadurch gekennzeichnet,** daß
der Schritt der Aussendung des Herzreizimpulses derart zeitlich gesteuert ausgeführt wird, daß das gleichzeitige oder überlagerte Auftreten einer spontanen Herzaktion und eines evozierten Potentials verhindert ist, wobei
im Falle einer atrium-synchronen Stimulation die Aussendung eines Herzreizimpulses im Ventrikel die Verzögerung zwischen der spontanen Atrium-Aktion und der Stimulation im Ventrikel kleiner als die natürliche AV-Überleitungszeit eingestellt wird und/oder
im Falle der ventrikelinhibierten Stimulation in einer ersten Stufe eine Folge mehrerer Herzreizimpulse mit einer ersten Rate, anschließend in einer zweiten Stufe eine zweite Folge von Herzreizimpulsen mit einer zweiten, gegenüber der ersten im wesentlichen sprunghaft vergrößerten Rate und anschließend in einer dritten Stufe eine dritte Folge von Herzreizimpulsen mit einer dritten, gegenüber der zweiten im wesentlichen sprunghaft wieder verringerten Rate ausgesandt wird, wobei während mindestens der ersten mit der dritten Rate abgegebenen Herzreizimpulse das gleichzeitige oder überlagerte Auftreten einer spontanen Herzaktion und eines evozierten Potentials verhindert ist.

2. Verfahren nach Anspruch 1, **dadurch gekennzeichnet,** daß im Falle der ventrikelinhibierten Stimulation der Impulsabstand in der dritten Stufe gleich dem Impulsabstand in der ersten Stufe gewählt wird.

3. Verfahren nach Anspruch 1 oder 2, **dadurch gekennzeichnet,** daß nach dem Schritt der Ausendung des Herzreizimpulses ein Schritt der Aufnahme und Auswertung eines Herzaktionspotentials in Abhängigkeit von der Zeit im Herzen ausgeführt wird.

4. Verfahren nach Anspruch 3, **dadurch gekennzeichnet,** daß der Schritt der Aufnahme und Auswertung die intraventrikuläre Aufnahme mehrerer Spannungswerte in Zeitabständen innerhalb eines im vorbestimmten Zeitraum festgelegten Meßzeitfensters und einen Vergleich mit einem vorbestimmten Signalverlauf, insbesondere unter Einschluß einer Amplitudendiskriminierung des Signals in der Zeit- oder Frequenzdomäne bezüglich mindestens einer vorbestimmten Referenzspannung mit anschließender Bewertung, zur Unterscheidung zwischen einer spontanen und einer stimulierten Herzaktion durch einen Signalmustervergleich einschließt.

5. Verfahren nach Anspruch 4, **dadurch gekennzeichnet,** daß die Frequenzanalyse mit anschließender Bewertung einen Amplitudenvergleich der Signalanteile des Herzaktionspotentials bei mindestens zwei vorbestimmten Frequenzen oder in mindestens zwei Frequenzbereichen einschließt.

6. Verfahren nach Anspruch 5, **dadurch gekennzeichnet,** daß die Amplitudendiskriminierung mit anschließender Bewertung eine Unterscheidung zwischen spontaner Herzaktion oder evoziertem Potential anhand der absoluten oder relativen Häufigkeit des Auftretens von Amplitudenwerten unterhalb der Referenzspannung einschließt.

7. Verfahren nach einem der Ansprüche 3 bis 6, **dadurch gekennzeichnet,** daß das aufgenommene Herzaktionspotential vor der Auswertung einer Bandfilterung mit einem Durchlaßbereich von 0,5 bis 10 Hz unterzogen wird.

8. Verfahren nach einem der Ansprüche 3 bis 7, **dadurch gekennzeichnet,** daß das Meßzeitfenster eine Breite von 60 ms hat und sein Beginn zwischen 150 und 250 ms nach dem Herzreizimpuls in Schritten einstellbar ist.

9. Verfahren nach einem der Ansprüche 3 bis 8, **dadurch gekennzeichnet,** daß die Abtastrate für die einzelnen Messungen innerhalb des Meßzeitfensters größer als 100 Hz ist.

10. Verfahren nach einem der Ansprüche 3 bis 9, **dadurch gekennzeichnet,** daß die Referenzspannung zwischen 1 und 15 mV in Schritten von 1 mV einstellbar ist.

11. Verfahren nach einem der vorangehenden Ansprüche, **dadurch gekennzeichnet,** daß die unterhalb der natürlichen AV-Überleitungszeit liegende AV-Verzögerung auf kleiner oder gleich 100 ms eingestellt wird.

12. Verfahren nach einem der Ansprüche 3 bis 11, **dadurch gekennzeichnet,** daß das aufgenommene und ausgewertete Herzaktionspotential zur Gewinnung einer ANS-Information zur Steuerung eines ratenadaptiven Schrittmachers verwendet wird.

13. Vorrichtung zur Durchführung Verfahrens nach einem der vorangehenden Ansprüche, **dadurch gekennzeichnet,** daß eine zeitlich steuerbare Vorrichtung (P; P') zur Erzeugung von Herzreizimpulsen, die Mittel (AVD') zur vorwählbaren Einstellung der AV-Verzögerung zwischen einem in das Atrium und einem in das Ventrikel ausgesandten Herzreizimpuls und/oder Mittel (RC') zur vorwählbaren Einstellung des Impulsabstandes aufweist, und !eine Steuereinheit (!b; MC, IC; MC') zur Steuerung des Verfahrensablaufes vorgesehen ist.

14. Vorrichtung nach Anspruch 13, **dadurch gekennzeichnet,** daß eine von einer Schrittmachereingangsschaltung (2) zur Erfassung eines intrakardialen EKG getrennte Aufnahme- und Auswertungsschaltung (1a; TF, TA, IC) vorgesehen ist.

15. Vorrichtung nach Anspruch 14, **dadurch gekennzeichnet,** daß die Aufnahme- und Auswertungsschaltung (1a, TF, TA, IC) und die Steuereinheit (1b; IC, MC) implantierbar sind und letztere von außerhalb des Körpers, insbesondere über einen Reed-Schalter (RS), aktivierbar ist.

16. Vorrichtung nach Anspruch 14 oder 15, **dadurch gekennzeichnet,** daß die Aufnahme- und Auswertungsschaltung (1a) aufweist:
- eine Austastschaltung (11) zum Ausblenden von Signalen unmittelbar nach dem Herzreizimpuls,
- einen Vorverstärker (12)
- einen programmierbaren Meßverstärker (13),
- ein Bandfilter (14) mit einem Durchlaßbereich von 0,5 bis 10 Hz,
- eine Vergleichereinheit (15) zur Amplitudendiskriminierung des Meßsignals,
- einen A/D-Wandler und
- eine logische Verarbeitungseinheit zur Analyse der Vergleichsergebnisse entsprechend dem Verfahrensablauf.

17. Vorrichtung nach einem der Ansprüche 13 bis 16, **dadurch gekennzeichnet,** daß Mittel (IA', IV') zur eigenständigen Erfassung der Herzreizimpulse und zur Triggerung des Verfahrensablaufes in Reaktion auf die erfaßten Herzreizimpulse vorgesehen sind.

18. Vorrichtung nach einem der Ansprüche 13 bis 17**, dadurch gekennzeichnet,** daß die Steuereinheit mindestens Teile der Steuereinheit (MC; MC') eines die Herzreizimpulse erzeugenden, extern programmierbaren Schrittmachers (P, P')) umfaßt.
